# EUROPEAN PATENT APPLICATION

(11) **EP 4 817 735 A1**
(43) Date of publication of application: **07.10.2026**
(21) Application number: 24897574.0
(22) Date of filing: 27.11.2024
(51) Int. Cl.: B01J 20/20, B01J 20/28, B01J 20/30, C01B 32/318

(54) **CARBONACEOUS MATERIAL, METHOD FOR PRODUCING SAME, AND ADSORPTION FILTER**

(30) Priority: 29.11.2023 JP 2023201660
(71) Applicant: Osaka Gas Chemicals Co., Ltd., Osaka-shi, Osaka 550-0023 (JP)
(72) Inventor: KOBAYASHI, Aya, Osaka-shi, Osaka 550-0023 (JP); FUJII, Noriko, Osaka-shi, Osaka 550-0023 (JP); SEIKE, Naoya, Osaka-shi, Osaka 550-0023 (JP); UEDA, Sakae, Osaka-shi, Osaka 550-0023 (JP); ASADA, Takuya, Osaka-shi, Osaka 550-0023 (JP)
(74) Representative: Marks & Clerk LLP
(86) International application number: PCT/JP2024/041935
(87) International publication number: WO 2025/115890

(57) **Abstract**

The carbonaceous material of the present invention has a pore volume (cm³/g) of 0.23 cm³/g or more and 0.35 cm³/g or less per g of the carbonaceous material for pores with a pore size of 0.80 nm or less, calculated from a nitrogen adsorption isotherm by a QSDFT method, a bulk density of 0.43 g/mL or more and 0.65 g/mL or less, measured in accordance with JIS K 1474 (2014), and a Reactive Black 5 value of 3.0 g/L or more and 60.0 g/L or less.

## Description

### TECHNICAL FIELD

The present invention relates to a carbonaceous material, a method for producing the same, and an adsorption filter.

### BACKGROUND ART

Volatile organic compounds (VOCs) in automobile exhaust gas not only contain harmful substances such as aldehydes but also cause harmful substances to be generated through photochemical reactions. Examples of the VOC which is discharged in a large amount and is likely to cause a photochemical reaction include butanes such as n-butane and isobutane, and butenes such as 1,2-butadiene and 1,3-butadiene (hereinafter, in the present specification, these butanes and butenes are also collectively referred to simply as "butanes"). Butanes enter the vehicle interior in a driving environment and cause adverse health effects on a driver as well as discomfort due to odor. Therefore, automobiles are usually equipped with an automotive filter incorporating activated carbon as an adsorbent for these substances.

As an adsorbent for such a filter, for example, Patent Document 1 describes a composite gas adsorbent in which predetermined amounts of aromatic aminosulfonic acid and specific organic acids are impregnated in activated carbon having a BET specific surface area of 700 m²/g or more and 1300 m²/g or less. The adsorbent of Patent Document 1 is intended to adsorb a composite gas containing aldehyde and butane.

### CITATION LIST

### PATENT DOCUMENT

Patent Document 1: Japanese Unexamined Patent Publication No. 2011-143359

### SUMMARY OF THE INVENTION

### TECHNICAL PROBLEM

The adsorbent of Patent Document 1 exhibits high aldehyde removal performance due to the impregnated substances regardless of the adsorption characteristics of the activated carbon, while exhibiting butane removal performance by controlling only the specific surface area, which is an adsorption characteristic of the activated carbon. However, the control of only the specific surface area cannot control small micropores effective for butane adsorption, and high butane removal performance cannot be exhibited.

The present invention has been made in view of such problems, and aims to provide a carbonaceous material having high adsorption performance for butanes, a method for producing the same, and an adsorption filter.

### SOLUTION TO THE PROBLEM

As a result of intensive studies to accomplish the aim described above, the present inventors have found that a carbonaceous material having a pore volume, a bulk density, and a Reactive Black 5 value in respective predetermined ranges exhibits high adsorption performance for butanes, thereby completing the present invention.

The present invention includes the following embodiments.
[1] A carbonaceous material having
   a pore volume (cm³/g) of 0.23 cm³/g or more and 0.35 cm³/g or less per g of the carbonaceous material for pores with a pore size of 0.80 nm or less, the pore volume being calculated from a nitrogen adsorption isotherm by a QSDFT method,
   a bulk density of 0.43 g/mL or more and 0.65 g/mL or less measured in accordance with JIS K 1474 (2014), and
   a Reactive Black 5 value of 3.0 g/L or more and 60.0 g/L or less.
[2] The carbonaceous material of [1], wherein an iodine adsorption number is 710 mg/g or more and 1,500 mg/g or less.
[3] The carbonaceous material of [1], wherein a percentage of the pore volume of pores with a pore size of 0.80 nm or less is 61% or more and 92% or less.
[4] The carbonaceous material of [1], wherein an average pore size of micropores is 0.60 nm or more and 0.80 nm or less.
[5] The carbonaceous material of any one of [1] to [4], wherein the carbonaceous material is used for adsorbing at least one selected from the group consisting of n-butane, isobutane, 1,2-butadiene, and 1,3-butadiene.
[6] A method for producing the carbonaceous material of any one of [1] to [4], the method including: carbonizing a raw material to obtain a carbide; and subjecting the carbide to an activation treatment to obtain an activated product.
[7] The method of [6], further including washing the activated product.
[8] The method of [6], wherein the raw material is coconut shells.
[9] An adsorption filter including the carbonaceous material of any one of [1] to [4].
[10] The adsorption filter of [9], wherein the carbonaceous material is used for an automobile.

### ADVANTAGES OF THE INVENTION

According to the present invention, a carbonaceous material having high adsorption performance for butanes, a method for producing the same, and an adsorption filter can be provided.

### BRIEF DESCRIPTION OF THE DRAWING

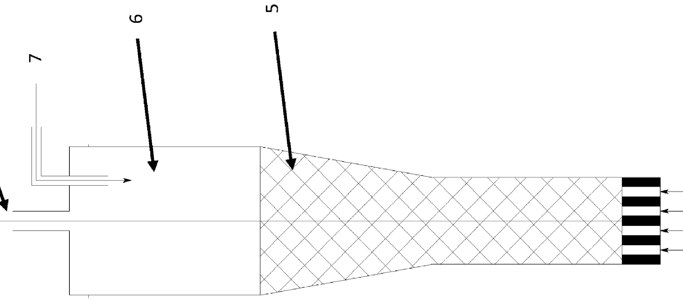

[FIG. 1] FIG. 1 is a schematic cross-sectional view of a fluidized bed furnace.

### DESCRIPTION OF EMBODIMENTS

An embodiment of the present invention (hereinafter simply referred to as "the present embodiment") will be described in detail below. The present embodiment described below is an example for explaining the present invention, and the present invention is not limited to the present embodiment.

### [Carbonaceous Material]

The carbonaceous material of the present embodiment has a pore volume (cm³/g) of 0.23 cm³/g or more and 0.35 cm³/g or less per g of the carbonaceous material for pores with a pore size of 0.80 nm or less, the pore volume being calculated from a nitrogen adsorption isotherm by a QSDFT method, a bulk density of 0.43 g/mL or more and 0.65 g/mL or less measured in accordance with JIS K 1474 (2014), and a Reactive Black 5 value of 3.0 g/L or more and 60.0 g/L or less.

The carbonaceous material satisfying such requirements has high adsorption performance for butanes. Therefore, the carbonaceous material of the present embodiment is suitable for an adsorption filter for removing butanes. Examples of the butanes include butanes such as n-butane and iso-butane, and butenes such as 1,2-butadiene and 1,3-butadiene.

In the carbonaceous material, the pore volume (cm³/g) of pores with a pore size of 0.80 nm or less per g of the carbonaceous material, calculated from a nitrogen adsorption isotherm by the QSDFT method (hereinafter, also simply referred to as "the pore volume of pores with a pore size of 0.80 nm or less") is 0.23 cm³/g or more and 0.35 cm³/g or less. The carbonaceous material has a pore volume of pores with a pore size of 0.80 nm or less within a specific range, and therefore has excellent adsorption performance for butanes having a relatively small molecular size. That is, when the pore volume of pores with a pore size of 0.80 nm or less is 0.23 cm³/g or more, a pore volume of small pores suitable for removing substances having relatively small molecular sizes is sufficient, and the adsorption performance for butanes is significantly improved. In addition, when the pore volume of pores with a pore size of 0.80 nm or less is 0.35 cm³/g or less, the percentage of pore volume of pores suitable for substances having smaller molecular sizes than those of butanes is reduced, and the pore volume of pores suitable for butanes is increased accordingly. This significantly improves the adsorption performance for butanes.

According to the classification standards of International Union of Pure and Applied Chemistry (IUPAC), the pores of a carbonaceous material described in the present specification are classified into micropores with a pore size below 2.0 nm, mesopores with a pore size of 2.0 nm or more and 50.0 nm or less, and macropores having a pore size larger than 50.0 nm. The micropores are pores smaller than mesopores, and are effective for adsorbing butanes having relatively small molecular sizes.

In the present specification, the pore volume of pores with a pore size of 0.80 nm or less is calculated by the quenched solid density functional theory (QSDFT) method. The QSDFT method is an analysis method for pore size analysis of geometrically and chemically irregular microporous and mesoporous carbons, and can calculate pore size distributions of pores of about 0.5 nm or more and about 40 nm or less. The QSDFT method clearly considers the effects of the roughness and non-uniformity of the pore surface, and thus is a technique that allows the pore size distribution analysis with greatly improved accuracy. The specific method for measuring and calculating the pore volume of the pores with a pore size of 0.80 nm or less is described in the Examples.

The pore volume of pores with a pore size of 0.80 nm or less is preferably 0.235 cm³/g or more and 0.300 cm³/g or less, more preferably 0.240 cm³/g or more and 0.270 cm³/g or less. When the range of the pore volume of the pores with a pore size of 0.80 nm or less is within the above range, a carbonaceous material having higher adsorption performance for butanes tends to be obtained.

The bulk density of the carbonaceous material measured in accordance with JIS K 1474 (2014) (hereinafter, also simply referred to as "bulk density") is 0.43 g/mL or more and 0.65 g/mL or less. The bulk density is greatly affected by the pore volume of the carbonaceous material. Therefore, when the bulk density of the carbonaceous material having a 50% particle size (D50), based on a volume-based cumulative distribution, of 9.0 µm to 11.0 µm is measured, the value of the bulk density serves as an index of the pore volume of the carbonaceous material. When the bulk density is within the above range, the carbonaceous material tends to be able to realize the adsorption performance for butanes at a higher level. When the bulk density is 0.43 g/mL or more, the pores of the carbonaceous material are not excessively large, and the carbonaceous material can have many pores effective for adsorption of butanes. When the bulk density is 0.65 g/mL or less, the carbonaceous material tends to have a sufficient number of pores contributing to adsorption of butanes. The specific method for measuring and calculating the bulk density is described in the Examples.

The bulk density is preferably 0.50 g/mL or more and 0.63 g/mL or less, more preferably 0.52 g/mL or more and 0.60 g/mL or less. When the range of the bulk density is within the above range, a carbonaceous material having higher adsorption performance for butanes tends to be obtained.

The Reactive Black 5 value of the carbonaceous material is 3.0 g/L or more and 60.0 g/L or less. Reactive Black 5 is a dye represented by the following formula (1), and is also referred to as C.I. Reactive Black-5.

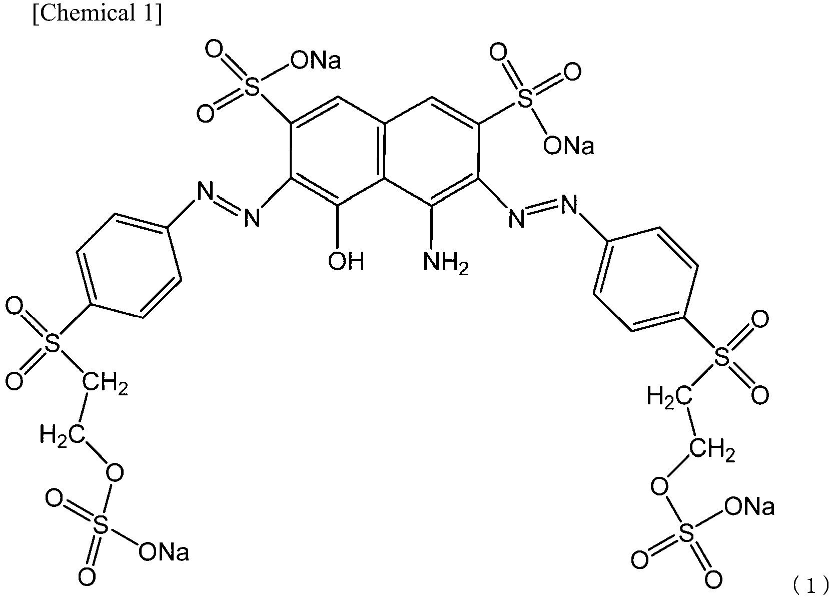

Reactive Black 5 has a large molecular weight of 995.88 and a bulky structure, and thus, the Reactive Black 5 value is used as an index of the cumulative pore volume for large pores of the carbonaceous material. When the Reactive Black 5 value is within the above range, the carbonaceous material exhibits high adsorption performance for butanes. When the Reactive Black 5 value is 3.0 g/L or more, relatively large pores that are less likely to adsorb butanes are reduced among the pores of the carbonaceous material. Therefore, the pore volume effective for adsorption of butanes increases, and as a result, the adsorption performance for butanes is significantly improved. In addition, when the Reactive Black 5 value is 60.0 g/L or less, the carbonaceous material can have a pore volume effective for adsorption of butanes. This significantly improves the adsorption performance for butanes.

The Reactive Black 5 value can be calculated, for example, as follows. Specifically, first, a test solution containing Reactive Black 5 and a residual solution obtained by mixing the carbonaceous material with the test solution to cause the carbonaceous material to sufficiently adsorb Reactive Black 5 and removing the carbonaceous material that adsorbed Reactive Black 5 are prepared, and the absorbance of each solution is measured with an ultraviolet and visible spectrophotometer at a wavelength of 594 nm and an optical path length (cell length) of 10 mm. Then, the residual ratio (%) of Reactive Black 5 contained in the residual solution and the adsorption amount (/g) of Reactive Black 5 per g of the carbonaceous material are calculated using the absorbances. Using these values, the Reactive Black 5 value (g/L) is calculated as the amount of the carbonaceous material required to remove 99% of Reactive Black 5 in a 1 L test solution. For the measurement of the Reactive Black 5 value, it is preferable to use a carbonaceous material having a 50% particle size (D50) in a volume-based cumulative distribution controlled to 9.0 µm or more and 11.0 µm or less. In the present specification, the 50% particle size (D50) refers to a value measured as a volume-based median particle size using a laser diffraction particle size analyzer. The specific method for measuring and calculating the Reactive Black 5 value is described in Examples.

The Reactive Black 5 value is preferably 10.0 g/L or more and 55.0 g/L or less, more preferably 15.0 g/L or more and 50.0 g/L or less, much more preferably 20.0 g/L or more and 40.0 g/L or less, still more preferably 22.0 g/L or more and 30.0 g/L or less. When the range of the Reactive Black 5 value is within the above range, a carbonaceous material having higher adsorption performance for butanes tends to be obtained.

The iodine adsorption number of the carbonaceous material is preferably 710 mg/g or more and 1,500 mg/g or less, more preferably 750 mg/g or more and 1,300 mg/g or less, much more preferably 800 mg/g or more and 1,120 mg/g or less. When the range of the iodine adsorption number is within the above range, a carbonaceous material having higher adsorption performance for butanes tends to be obtained.

The iodine adsorption number is an index of the surface area of the pores of the carbonaceous material capable of physically adsorbing the butanes. When the iodine adsorption number of the carbonaceous material is within the above range, the carbonaceous material exhibits high adsorption performance for butanes. When the iodine adsorption number is 710 mg/g or more, the pore volume of the carbonaceous material does not become too small, and the carbonaceous material can have many pores effective for adsorption of butanes. When the iodine adsorption number is 1,500 mg/g or less, the pores of the carbonaceous material are not excessively large, and the carbonaceous material can have many pores effective for adsorption of butanes.

The iodine adsorption number is measured and calculated in accordance with JIS K 1474 (2014). The specific method for measuring and calculating the iodine adsorption number is described in Examples.

In the carbonaceous material, the percentage of the pore volume of pores with a pore size of 0.80 nm or less is preferably 61% or more and 92% or less, more preferably 70% or more and 90% or less, even more preferably 73% or more and 89% or less. When the percentage of the pore volume is within the above range, a carbonaceous material having higher adsorption performance for butanes tends to be obtained.

The percentage of the pore volume of pores with a pore size of 0.80 nm or less refers to a percentage of small pores of the carbonaceous material. When the percentage of the pore volume of pores with a pore size of 0.80 nm or less is 61% or more, the carbonaceous material has many small pores effective for butanes and tends to exhibit high adsorption performance. When the percentage of the pore volume of pores with a pore size of 0.80 nm or less is 92% or less, the pore size does not become excessively small, and the carbonaceous material has many pores effective for adsorption of butanes, and thus, the carbonaceous material tends to exhibit high adsorption performance.

The percentage of the pore volume of pores with a pore size of 0.80 nm or less is determined by the ratio of the pore volume of pores with a pore size of 0.80 nm or less to the pore volume of pores with a pore size of 2.0 nm or less. The specific method for measuring and calculating the pore volume of pores with a pore size of 0.80 nm or less is described in the Examples.

The average pore size of micropores of the carbonaceous material is preferably 0.60 nm or more and 0.80 nm or less, preferably 0.61 nm or more and 0.75 nm or less, more preferably 0.63 nm or more and 0.70 nm or less. When the range of the average pore size is within the above range, a carbonaceous material having higher adsorption performance for butanes tends to be obtained.

The average pore size of micropores refers to an average value of the pore sizes of micropores in the carbonaceous material. When the average pore size is within the above range, the carbonaceous material tends to more suitably have pores effective for adsorption of butanes.

The average pore size of the micropores is calculated by the following equation (2) using the specific surface area (m²/g) of micropores with a pore size of 2.0 nm or less among micropores per g of the carbonaceous material, calculated from the nitrogen adsorption isotherm by the QSDFT method (hereinafter also merely referred to as "specific surface area of micropores") and the pore volume (m³/g) of micropores with a pore size of 2.0 nm or less among micropores per g of the carbonaceous material, calculated from the nitrogen adsorption isotherm by the QSDFT method (hereinafter also merely referred to as "pore volume of micropores"). The specific method for measuring and calculating the average pore size is described in the Examples. In measuring and calculating the average pore size of the micropores, the micropores included the pores with a pore size of 2.0 nm. 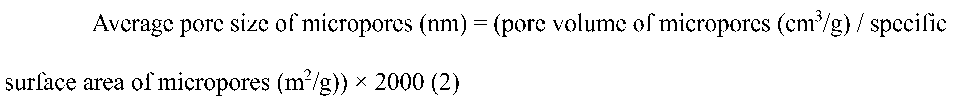 Average pore size of micropores (nm) = (pore volume of micropores (cm3/g) / specific surface area of micropores (m2/g)) × 2000 (2)

The shape of the carbonaceous material varies depending on the application and is not particularly limited. The shape includes, for example, powdered, lumpy, crushed, spherical, cylindrical, ellipsoidal, distorted, elliptical, elliptical pyramidal, and rod shapes including polygonal columns such as triangular, square, pentagonal, and hexagonal columns, thread shapes, pellet shapes such as solid and hollow pellets, crushed shapes such as powdered shapes, substrates (sheets), woven fabric (cloths), fibrous shapes such as felt, and block shapes.

The carbonaceous material preferably has a shape that makes the carbonaceous material applicable to a known adsorption filter. The shape includes, for example, spherical, ellipsoidal, distorted, rod shapes, thread shapes, pellet shapes, crushed shapes such as powdered shapes, substrates (sheets), woven fabric (cloths), fibrous shapes, and block shapes. These shapes can be appropriately selected according to the specific application. Among them, the carbonaceous material is preferably crushed, more preferably powdered, due to high adsorption performance per unit volume. The powdered carbonaceous material may have any size, and the particle size may be appropriately adjusted according to the specific application.

In the present specification, the term "crushed" refers to particles of any shape that is not uniform and is usually angular. The term "powdered" refers to, for example, fine powder, powder, fine particles, and granular powder, and the 50% particle size (D50) in the volume-based cumulative distribution is generally 1 µm or more and 150 µm or less.

For example, when the carbonaceous material is used as an automotive adsorption filter, the shape thereof is preferably powdered, granular, pellet, or a fiber shape. When the carbonaceous material has such a shape, it is more easily subjected to sheet processing and pleat filling processing, and a suitable filter can be obtained. Therefore, the carbonaceous material tends to be less likely to flow out from the obtained filter and can be suitably used as an adsorption filter.

The carbonaceous material is preferably activated carbon.

### [Method for Producing Carbonaceous Material]

The carbonaceous material of the present embodiment can be obtained by a known production method.

Examples of such a method include a thermal decomposition method, an activation method, a coating method, and a vapor deposition method. The activation method is preferably used as the production method. Use of these production methods tends to allow even easier production of a carbonaceous material having a pore volume (cm³/g) of 0.23 cm³/g or more and 0.35 cm³/g or less per g of the carbonaceous material for pores with a pore size of 0.80 nm or less, calculated from a nitrogen adsorption isotherm by a QSDFT method, a bulk density of 0.43 g/mL or more and 0.65 g/mL or less measured in accordance with JIS K 1474 (2014), and a Reactive Black 5 value of 3.0 g/L or more and 60.0 g/L or less.

The method for producing the carbonaceous material of the present embodiment includes carbonizing a raw material to obtain a carbide and subjecting the carbide to an activation treatment to obtain an activated product. The method for producing the carbonaceous material of the present embodiment preferably includes washing the activated product.

### (Carbonization)

The method for producing a carbonaceous material includes carbonizing a raw material to obtain a carbide.

Any raw material can be used as long as it is a material from which a desired carbonaceous material can be obtained. Examples of the raw material include plant-based or fossil-based raw materials such as wood, wood flour, fruit shells such as coconut shells, seeds such as palm kernels, plum pits, and peach pits, by-products in pulp production, bagasse, blackstrap molasses, coal (such as peat, lignite, brown coal, and bituminous coal), anthracite, petroleum distillation residues, petroleum pitch, coke, and coal tar; various synthetic resins such as phenol resin, vinyl chloride resin, vinyl acetate resin, melamine resin, urea resin, resorcinol resin, celluloid, epoxy resin, polyurethane resin, polyester resin, acrylic resin, and polyamide resin; synthetic rubbers such as polybutylene, polybutadiene, and polychloroprene; other synthetic wood; and synthetic pulp. These raw materials may be used alone, or two or more of them may be used in combination at any ratio according to the required specifications.

The raw material is preferably a natural product, more preferably the coconut shells. Use of such a raw material tends to allow easier production of a carbonaceous material having a pore volume (cm³/g) of 0.23 cm³/g or more and 0.35 cm³/g or less per g of the carbonaceous material for pores with a pore size of 0.80 nm or less, calculated from a nitrogen adsorption isotherm by a QSDFT method, a bulk density of 0.43 g/mL or more and 0.65 g/mL or less measured in accordance with JIS K 1474 (2014), and a Reactive Black 5 value of 3.0 g/L or more and 60.0 g/L or less.

The raw material may contain additives or any other substances as necessary. The additives or any other substances may be added to the carbide as necessary.

Examples of such additives include water, coal tar, anhydrous tar, hard pitch, coal tarbased pitch, and petroleum-based pitch. The additives may be used alone, or two or more of them may be used in combination.

The additives are each blended in an amount of usually 1.0 part by mass or more and 50.0 parts by mass or less relative to 100 parts by mass of the raw material or the carbide. The total amount of the additives is usually 1 part by mass or more and 100 parts by mass or less relative to 100 parts by mass of the raw material or the carbide. When the raw material or the carbide is mixed with the additive, the oxygen amount of the raw material or the carbide may be controlled in advance within the range of 1.0 mass% or more and 20.0 mass% or less relative to 100 mass% of the raw material or the carbide, as necessary. The oxygen amount can be controlled by, for example, mixing the raw material or the carbide with oxygen under heating at 150°C or more and 300°C or less.

The method for producing the carbonaceous material may include pulverizing or molding the raw material before carbonizing the raw material. Examples of this method include a method of pulverizing the raw material into a powder form or a granular form with a known pulverizer before the carbonization, and then carbonizing the pulverized material. Another method includes molding the raw material into pellets by a known technique before carbonizing the raw material, and then carbonizing the pellets.

When the raw material is powdered, the particle size of the powder (50% particle size in a volume-based cumulative distribution, D50) is preferably 1 µm or more and 150 µm or less. When the shape of the raw material is granular, the particle size (D50) is preferably 150 µm or more and 2000 µm or less. When the raw material is formed into pellets, the particle size (D50) of the pellets is preferably 2000 µm or more and 3000 µm or less.

The raw material may be carbonized by any method, for example, by heating the raw material to 300°C or higher and 900°C or lower, preferably 400°C or higher and 800°C or lower, under oxygen-free conditions.

The carbonization time can be appropriately set depending on the raw material and the facility for carbonization. The carbonization time is, for example, 15 minutes or more and 20 hours or less, preferably 30 minutes or more and 10 hours or less. The carbonization can be performed using a known production equipment such as a fluidized bed furnace. The carbonization may be performed at a pressure reduced by evacuation or in a nitrogen atmosphere.

The method for producing the carbonaceous material may include pulverizing the carbide into a powder form or a granular form with a known pulverizer. The carbide may be formed into pellets by a known method. These production methods tend to allow even easier production of a carbonaceous material having a pore volume (cm³/g) of 0.23 cm³/g or more and 0.35 cm³/g or less per g of the carbonaceous material for pores with a pore size of 0.80 nm or less, calculated from a nitrogen adsorption isotherm by a QSDFT method, a bulk density of 0.43 g/mL or more and 0.65 g/mL or less measured in accordance with JIS K 1474 (2014), and a Reactive Black 5 value of 3.0 g/L or more and 60.0 g/L or less. In the method for producing a carbonaceous material, the carbide may be pulverized into powder or granules, or molded into pellets, and then, if necessary, an additive or the like may be added to the powdered carbide, followed by kneading by a known method, and the resulting kneaded product may be molded by a known method.

When the carbide is in the form of powder, granules, or pellets, the preferred range of the particle size (50% particle size in volume-based cumulative distribution, D50) of the carbide is the same as the preferred range when the raw material is in the form of powder, granules, or pellets.

In the method for producing a carbonaceous material, a carbide, a powdered or granulated carbide, a kneaded product, or a powdered or granular kneaded product may be molded into cylindrical pellets by using a known method. This tends to allow even easier production of a carbonaceous material having a pore volume (cm³/g) of 0.23 cm³/g or more and 0.35 cm³/g or less per g of the carbonaceous material for pores with a pore size of 0.80 nm or less, calculated from a nitrogen adsorption isotherm by a QSDFT method, a bulk density of 0.43 g/mL or more and 0.65 g/mL or less measured in accordance with JIS K 1474 (2014), and a Reactive Black 5 value of 3.0 g/L or more and 60.0 g/L or less.

When the carbide is molded into the cylindrical pellets, the diameter of the cylindrical pellet is preferably 0.1 mm or more and 4.0 mm or less. The aspect ratio (diameter:height) of the cylindrical pellet is preferably 1:1 to 1:10.

The carbonization provides a carbide as the raw material.

The method for producing the carbonaceous material may include washing and/or drying the carbide after the carbonization. The conditions for these steps are not particularly limited, and known conditions can be employed. Reference may also be made to the washing and drying described below.

### (Activation)

The method for producing the carbonaceous material includes subjecting the carbide to an activation treatment to obtain an activated product.

Any known method can be used to perform the activation treatment.

For the activation treatment, known production equipment such as a rotary kiln, a fluidized bed furnace, and a sleeve furnace (vertical furnace) can be used. The activation treatment may be performed at a pressure reduced by evacuation or in a nitrogen atmosphere.

The activation treatment is preferably performed using a fluidized bed furnace. Since the fluidized bed furnace can efficiently bring the carbide into contact with the activating gas, mesopores tend not to develop, while the carbonaceous material contains a large number of micropores, and in particular, pores with a pore size of 0.80 nm or less can be provided efficiently in the carbonaceous material in a short time.

When the activation treatment is performed using the fluidized bed furnace, the carbide to be charged in the fluidized bed furnace preferably has a particle size retained on a 70 mesh sieve (aperture size: 243 µm) and passing through a 10 mesh sieve (aperture size: 1.54 mm), more preferably a particle size retained on a 70 mesh sieve (aperture size: 243 µm) and passing through a 14 mesh sieve (aperture size: 1.31 mm), in accordance with JIS Z 8801-1:2019 specifying test sieves of metal wire cloth. When the particle size of the carbide is within the above range, the activated product that has its mass reduced with the progress of the activation of the carbide can remain in the fluidized bed furnace, allowing more effective activation. As the carbide, a carbide having a particle size adjusted by cutting a raw material into a desired size before the carbonization may be used, or a carbide having a particle size adjusted by crushing and classifying the carbide into a desired size may be used.

Examples of the method of the activation treatment include a method using an activating gas such as a water vapor gas, an oxygen gas, and a carbon dioxide gas. By using an activating gas as the activation method, a carbonaceous material having a large number of micropores, particularly a larger number of pores with a pore size of 0.80 nm or less, tends to be more easily obtained. An inert gas such as nitrogen may be used in combination with the activating gas.

As the activating gas, it is preferable that one or more gas selected from the group consisting of water vapor gas, oxygen gas, carbon dioxide gas is used, and it is more preferable that all the water vapor gas, the oxygen gas, and the carbon dioxide gas are used.

The water vapor gas tends to achieve a sufficiently high reaction rate and allow the control of the reaction rate without decreasing the production efficiency. In addition, by using the water vapor gas, the carbonaceous material tends to have a large number of micropores, particularly a high percentage of micropores with a pore size of 0.80 nm or less. This tends to allow suitable production of a carbonaceous material having higher adsorption performance for butanes.

The activation reaction is usually an endothermic reaction, and thus, a certain amount or more of heat is required to allow the activation reaction to proceed more efficiently. The oxygen gas and the water vapor gas are preferably used together as the activating gas to maintain the heat. During the activation reaction, a volatile gas is generated and reacts with the oxygen gas to burn, keeping the amount of heat required for the activation. Examples of the volatile gas include combustible gases such as a hydrogen gas and a carbon monoxide gas generated by activating the carbide.

When an excessive amount of oxygen gas is introduced as the activating gas into the fluidized bed furnace, extra oxygen gas that does not react with the volatile gas is generated. The excess oxygen gas may undergo a combustion reaction with the carbide, destroying the pores of the carbonaceous material. Therefore, the activation treatment is preferably performed while controlling the amount of oxygen gas within a suitable range.

When water vapor gas and oxygen gas are used as the activating gas, the ratio of the water vapor gas is preferably 5 vol% or more and 15 vol% or less, more preferably 8 vol% or more and 12 vol% or less, relative to 1 vol% of the oxygen gas. The ratio within the above range tends to allow even easier production of a carbonaceous material having a pore volume (cm³/g) of 0.23 cm³/g or more and 0.35 cm³/g or less per g of the carbonaceous material for pores with a pore size of 0.80 nm or less, calculated from a nitrogen adsorption isotherm by a QSDFT method, a bulk density of 0.43 g/mL or more and 0.65 g/mL or less measured in accordance with JIS K 1474 (2014), and a Reactive Black 5 value of 3.0 g/L or more and 60.0 g/L or less.

When water vapor gas, oxygen gas, and carbon dioxide gas are used as the activating gas, the partial pressure of the water vapor gas is preferably 10 vol% or more and 30 vol% or less, more preferably 15 vol% or more and 25 vol% or less. The partial pressure of the oxygen gas is preferably 0.5 vol% or more and 5 vol% or less, more preferably 1 vol% or more and 4 vol% or less. The partial pressure of the carbon dioxide gas is preferably 1 vol% or more and 10 vol% or less, more preferably 3 vol% or more and 7 vol% or less. Note that an inert gas such as nitrogen may be contained as another gas. In this case, the partial pressure of the inert gas is preferably 55 vol% or more and 88.5 vol% or less, more preferably 64 vol% or more and 81 vol% or less. The ratio within the above range tends to allow even easier production of a carbonaceous material having a pore volume (cm³/g) of 0.23 cm³/g or more and 0.35 cm³/g or less per g of the carbonaceous material for pores with a pore size of 0.80 nm or less, calculated from a nitrogen adsorption isotherm by a QSDFT method, a bulk density of 0.43 g/mL or more and 0.65 g/mL or less measured in accordance with JIS K 1474 (2014), and a Reactive Black 5 value of 3.0 g/L or more and 60.0 g/L or less.

When the water vapor gas and the oxygen gas are used as the activating gas, the flow rate of the water vapor gas and the oxygen gas is preferably 10 liters (L) or more and 300 liters (L) or less in total per minute.

The time for the activation treatment can be appropriately set depending on the conditions such as the type of the raw material, the activation temperature, and the production equipment. The activation time is, for example, 20 minutes or more and 48 hours or less, preferably 30 minutes or more and 36 hours or less, more preferably 40 minutes or more and 24 hours or less, much more preferably 45 minutes or more and 480 minutes or less, and still more preferably 50 minutes or more and 360 minutes or less. The activation time within the above range tends to allow even easier production of a carbonaceous material having a pore volume (cm³/g) of 0.23 cm³/g or more and 0.35 cm³/g or less per g of the carbonaceous material for pores with a pore size of 0.80 nm or less, calculated from a nitrogen adsorption isotherm by a QSDFT method, a bulk density of 0.43 g/mL or more and 0.65 g/mL or less measured in accordance with JIS K 1474 (2014), and a Reactive Black 5 value of 3.0 g/L or more and 60.0 g/L or less.

The temperature at which the activation treatment is performed is preferably 750°C or higher and 1,200°C or lower, more preferably 800°C or higher and 1,100°C or lower, even more preferably 870°C or higher and 950°C or lower. The activation temperature within the above range tends to allow even easier production of a carbonaceous material having a pore volume (cm³/g) of 0.23 cm³/g or more and 0.35 cm³/g or less per g of the carbonaceous material for pores with a pore size of 0.80 nm or less, calculated from a nitrogen adsorption isotherm by a QSDFT method, a bulk density of 0.43 g/mL or more and 0.65 g/mL or less measured in accordance with JIS K 1474 (2014), and a Reactive Black 5 value of 3.0 g/L or more and 60.0 g/L or less.

Examples of an activation apparatus for performing the activation treatment include a fluidized bed furnace as shown in the schematic cross-sectional view of FIG. 1.

As shown in FIG. 1, the fluidized bed furnace usually includes gas inlets 1 to 4 in the lowermost layer, a fluidized bed 5 disposed above the gas inlets 1 to 4, a combustible gas combustion layer 6 disposed above the fluidized bed 5, and a gas outlet 8 disposed above the combustion layer 6. The gas introduced through the gas inlets 1 to 4 is sent toward the main direction A of the gas, and is discharged through the gas outlet 8 while passing through the fluidized bed 5 and the combustible gas combustion layer 6. A carbide as a raw material is charged into the fluidized bed 5 and activated in the fluidized bed 5, whereby a carbonaceous material is produced.

The gas inlets 1 to 4 are inlets for introducing each of the water vapor gas, the oxygen gas, the carbon dioxide gas, and the nitrogen gas into the fluidized bed furnace. The gas inlets 1 to 4 are preferably an inlet for water vapor gas, an inlet for carbon dioxide gas, an inlet for nitrogen gas, and an inlet for oxygen gas, respectively. By arranging the gas inlets 1 to 4 in this manner and introducing the gases into the fluidized bed furnace, the activation treatment of the carbide (raw material) tends to proceed more suitably, and the desired carbonaceous material tends to be produced more easily. Although four gas inlets are provided in FIG. 1, the number of gas inlets can be appropriately set depending on, for example, the type of raw material, the degree of activation, the size of the facility, and the like. The type of gas introduced into the gas inlets 1 to 4 can also be set as appropriate depending on, for example, the type of raw material, the degree of activation, the size of the facility, and the like.

As shown in FIG. 1, the fluidized bed furnace preferably includes an oxygen-containing gas inlet 7 for introducing an oxygen-containing gas. By introducing the oxygen-containing gas into the fluidized bed furnace through the oxygen-containing gas inlet 7, the volatile gas can be efficiently combusted. This generates combustion heat, which can further increase the temperature in the fluidized bed 5, and activation more efficiently proceeds.

In addition, since the volatile gas is efficiently combusted with the oxygen-containing gas, the oxygen gas introduced through the gas inlets 1 to 4 is less likely to be excessive. As a result, the oxygen gas can more suitably suppress the combustion reaction with the carbide, and therefore, the pores of the carbonaceous material can be more easily controlled.

For such reasons, a carbonaceous material having desired pores and a desired specific surface area tends to be produced more easily.

The oxygen-containing gas inlet 7 is preferably positioned in the fluidized bed furnace so as not to contact the upper end of the fluidized bed 5 and so as to introduce the oxygen-containing gas into the combustible gas combustion layer 6. The oxygen-containing gas inlet 7 is preferably oriented parallel to the main direction A of the gas and opposite to the main direction A of the gas (i.e., toward the downstream layer). By arranging the oxygen-containing gas inlet 7 in this manner, the oxygen-containing gas is suitably blown into the combustible gas combustion layer 6 of the combustible gas, and therefore, the temperature in the fluidized bed furnace can be further increased. This tends to allow the carbide to be more uniformly activated, and thus, a carbonaceous material having a desired pore size distribution to be produced more easily.

The material of the fluidized bed furnace is not particularly limited as long as it is a material used for a fluidized bed furnace, and examples thereof include stainless steel.

Use of the fluidized bed furnace as an activation apparatus tends to allow even easier production of a carbonaceous material having a pore volume (cm³/g) of 0.23 cm³/g or more and 0.35 cm³/g or less per g of the carbonaceous material for pores with a pore size of 0.80 nm or less, calculated from a nitrogen adsorption isotherm by a QSDFT method, a bulk density of 0.43 g/mL or more and 0.65 g/mL or less measured in accordance with JIS K 1474 (2014), and a Reactive Black 5 value of 3.0 g/L or more and 60.0 g/L or less.

The activation described above provides an activated product.

The method for producing a carbonaceous material may include washing and/or drying the activated product after the activation. The conditions for these steps are not particularly limited, and known conditions can be employed. Reference may also be made to the washing and drying described below.

### (Washing)

The carbonaceous material is preferably obtained through the washing the activated product obtained by the activation. The washing is more preferably carried out with water. When the carbonaceous material is subjected to the washing, a carbonaceous material having a pore volume (cm³/g) of 0.23 cm³/g or more and 0.35 cm³/g or less per g of the carbonaceous material for pores with a pore size of 0.80 nm or less, the pore volume being calculated by a QSDFT method from a nitrogen adsorption isotherm, a bulk density of 0.43 g/mL or more and 0.65 g/mL or less measured in accordance with JIS K 1474 (2014), and a Reactive Black 5 value of 3.0 g/L or more and 60.0 g/L or less tends to be produced more easily.

The washing temperature and time may be appropriately set to obtain the target carbonaceous material.

### (Drying)

The carbonaceous material is preferably obtained through the drying the washed product obtained by the washing.

Any known drying method such as natural drying, heat drying, or hot air drying can be used. The drying is preferably carried out under heating and/or reduced pressure. The drying under heating is preferably carried out by hot air because the product is dried evenly and stably. The drying is preferably performed until the moisture content of the carbonaceous material reaches 20.0 mass% or less, more preferably 10.0 mass% or less.

Examples of the heating method include a heating method using a stationary constant-temperature dryer, a stationary hot-air dryer, a vacuum dryer, a rotary evaporator, or an agitated dryer such as a conical dryer and a Nauta dryer. The heating may be performed at any temperature at which the carbonaceous material is cured and is not melted, preferably, for example, 40°C or more and 300°C or less.

The method for reducing the pressure can be, for example, a method using an oil pump, an oilless pump, an aspirator, or the like. The pressure in the pressure reduction method is generally 0.00001 MPa or more and 0.05 MPa or less.

The drying time is usually about 1 minute or more and 20 hours or less, although it depends on the drying temperature.

The carbonaceous material thus obtained may be used as it is, or may optionally be subjected to particle size adjustment by crushing, pulverization, and classification, purification by additional washing using, for example, water, an organic solvent, an aqueous acid solution, or an aqueous alkali solution, and additional heat treatment for imparting durability and structure adjustment as necessary, by known methods.

### [Applications]

The carbonaceous material can be suitably used for applications for removal, adsorption, concentration, and recovery of butanes. The applications may include a combination of removal, adsorption, concentration, and recovery as appropriate. Such applications include, for example, adsorption filters, and packed columns.

The carbonaceous material is suitably used for adsorbing at least one selected from the group consisting of n-butane, isobutane, 1,2-butadiene, and 1,3-butadiene. The carbonaceous material is more suitably used for adsorption of at least one selected from the group consisting of n-butane and isobutane, and is further suitably used for adsorption of n-butane.

### [Method for Adsorbing Butanes]

The method for adsorbing butanes includes adsorbing butanes in a carbonaceous material. Examples of the method for adsorbing butanes include a method in which butanes are adsorbed in the carbonaceous material to concentrate the butanes in the carbonaceous material. The method for the concentration may include the same steps as those of a known adsorption method, concentration method, recovery method, and the like for butanes, in addition to using the carbonaceous material of the present embodiment as the carbonaceous material.

In the adsorption, for example, the butanes are brought into contact with the carbonaceous material to adsorb the butanes in the carbonaceous material.

### [Apparatus]

The apparatus includes the carbonaceous material. The apparatus may have the same configuration as a known apparatus except that the carbonaceous material of the present embodiment is used as the carbonaceous material.

The function of the carbonaceous material is used by the apparatus including the carbonaceous material. The apparatus is preferably a treatment apparatus. In the present specification, the "treatment apparatus" may be any apparatus capable of removing, adsorbing, concentrating, and recovering butanes contained in VOC or the like by using the carbonaceous material of the present embodiment. Such a treatment apparatus may perform the removal, the adsorption, the concentration, and the recovery in combination as appropriate. Examples of the treatment apparatus include an apparatus having an adsorption filter, a column, a tank or bath, a tube, a cartridge, a cylinder, and a sheet each including the carbonaceous material (they may be hereinafter referred to as "a filter including the carbonaceous material"), an adsorption apparatus, and a concentration apparatus.

The apparatus includes, for example, an adsorption unit for bringing the butanes and the carbonaceous material into contact with each other. The adsorption unit may contain an adsorbent other than the carbonaceous material of the present embodiment, as necessary. Examples of the adsorbent include activated carbon other than the carbonaceous material of the present embodiment, zeolite, silica gel, activated alumina, nonwoven fabric, and porous organic compounds.

The treatment apparatus may include other adsorption filters together with the adsorption filter containing the carbonaceous material. Examples of the other adsorption filters include metal filters made of stainless steel, aluminum, bronze, copper, titanium, and nickel; and resin filters made of polypropylene, polyvinyl chloride, polyvinylidene chloride, polyethylene, polyamide, and fluorine-based resin.

The treatment apparatus may be a batch treatment apparatus or a continuous treatment apparatus, and the carbonaceous material can be used in either apparatus.

### [Adsorption Filter]

The adsorption filter of the present embodiment includes the carbonaceous material of the present embodiment. The adsorption filter is preferably used for an automobile.

Since the adsorption filter contains the carbonaceous material, it has high adsorption performance for butanes. Therefore, for example, by installing the adsorption filter in an automobile, it is possible to efficiently remove butanes floating in the vehicle interior.

The adsorption filter preferably includes the carbonaceous material and a fibrous binder.

Examples of the fibrous binder include a binder that can be fibrillated to be shaped with the carbonaceous material entangled therein. Such a fibrous binder can be either synthetic or natural. Examples of the fibrous binder include acrylic fibers, polyethylene fibers, polypropylene fibers, polyacrylonitrile fibers, cellulose fibers, nylon fibers, aramid fibers, and pulp.

The fibrous binder may be used alone, or two or more of them may be used in combination. The fibrous binder is preferably polyacrylonitrile fiber and/or pulp. Use of these fibrous binders can improve the density and strength of the adsorption filter and minimize performance degradation.

Since the carbonaceous material having higher adsorption performance for butanes tends to be obtained, the adsorption filter contains a fibrous binder in an amount of preferably 20 parts by mass or less, more preferably 10 parts by mass or less, relative to 100 parts by mass of the carbonaceous material. The lower limit is generally 0.01 parts by mass or more.

When the adsorption filter contains other functional components described later, the phrase "relative to 100 parts by mass of the carbonaceous material" regarding the filter composition may be replaced with "relative to 100 parts by mass of the total of the carbonaceous material and the other functional components."

The adsorption filter may contain other functional components as long as they do not impair the advantages of the present embodiment. Examples of the other functional components include: lead adsorbents such as titanosilicate and zeolite-based powders capable of adsorbing and removing soluble lead; ion exchange resins; chelate resins; and various adsorbents containing silver ions and/or silver compounds for imparting antibacterial properties.

### [Adsorption Filter for Automobile]

The adsorption filter for an automobile according to the present embodiment includes the carbonaceous material of the present embodiment. The adsorption filter for an automobile may have the same configuration as a known adsorption filter for an automobile except for containing the carbonaceous material of the present embodiment. The adsorption filter for an automobile containing the carbonaceous material has high adsorption performance for butanes. Therefore, for example, by installing the adsorption filter in an automobile, it is possible to efficiently remove butanes present in the vehicle interior.

### Examples

The present invention will be described in more detail with reference to examples and comparative examples, but the present invention is not limited to the examples.

### [Evaluation Method]

### (1) Pore Volume of Pores with Pore Size of 0.80 nm or less

### · Determination of Nitrogen Gas Adsorption Isotherm with BELSORP-MAX

A specific surface area/pore size distribution analyzer (BELSORP (registered trademark)-MAX (trade name) manufactured by MicrotracBEL Corp.) was used to heat the carbonaceous material under vacuum at 300°C for 3 hours, and then a nitrogen adsorption isotherm was measured at a temperature of 77K.

### · Determination of Pore Volume

The pore volume (cm³) of the micropores with a pore size of 0.80 nm or less per g of the carbonaceous material was calculated from the nitrogen gas adsorption isotherm by the QSDFT method in the following manner. Specifically, the pore size distribution was calculated by using the values of the nitrogen adsorption isotherm obtained in the above section "Determination of Nitrogen Gas Adsorption Isotherm with BELSORP-MAX" and applying N₂ at 77K carbon [slit pore / cyl. pore (QSDFT Ads. model)] as a calculation model, and thus the pore volume (cm³/g) of the micropores with a pore size of 0.80 nm or less was calculated.

### (2) Bulk Density

The bulk density (g/mL) of the carbonaceous material was measured in accordance with JIS K 1474 (2014). Specifically, first, the carbonaceous material was dried in a constant-temperature drier (DVS402 (trade name) manufactured by Yamato Scientific Co., Ltd.) at 115±5°C for 3 hours. Then, the dried product was allowed to cool to room temperature in a desiccator using silica gel as a drying agent to obtain a cooled carbonaceous material. The cooled carbonaceous material was introduced into a storage hopper of a bulk density measurement container (TD-V5 (trade name), manufactured by Toyo Denji Kikai Seisakusho), and using an attached vibrator, the bulk density measurement container was filled with the carbonaceous material up to the 100-mL mark. The mass of the carbonaceous material after filling was measured to the nearest 0.1 g.

### (3) Reactive Black 5 Value

The Reactive Black 5 value (g/L) of the carbonaceous material was measured.

Specifically, first, the carbonaceous material was pulverized so that the 50% particle size (D50) in the volume-based cumulative distribution was about 10.0 µm or less, and dried in a constant-temperature drier (DVS402 (trade name) manufactured by Yamato Scientific Co., Ltd.) at 115°C for 3 hours. Then, the dried product was allowed to cool to room temperature in a desiccator using silica gel as a drying agent to obtain a cooled carbonaceous material.

A test solution A containing a phosphate buffer and Reactive Black 5 (manufactured by Sigma-Aldrich) was prepared in the following manner. Specifically, first, 7.26 g of potassium dihydrogen phosphate (manufactured by FUJIFILM Wako Pure Chemical Corporation) and 28.66 g of disodium hydrogen phosphate dodecahydrate (manufactured by FUJIFILM Wako Pure Chemical Corporation) were dissolved in 2 L of distilled water to prepare a phosphate buffer (pH: 7.0). Thereafter, Reactive Black 5 was added in an amount of about 0.5 g to 1.2 g to 1 L of the resulting phosphate buffer to prepare the test solution A. In this case, the amount of Reactive Black 5 was controlled as follows. Specifically, the amount of Reactive Black 5 added to 1 L of the phosphate buffer was appropriately controlled so that the absorbance of the resulting test solution A diluted 20-fold with distilled water was 1.18 or more and 1.23 or less. The absorbance was measured at a wavelength of 594 nm using a glass cell with an optical path length of 10 mm and an ultraviolet and visible spectrophotometer (double-beam spectrophotometer U-2910 (trade name) manufactured by Hitachi High-Tech Corporation). The 20-fold diluted solution of the test solution A obtained as described above was used as a test solution B for the following absorbance measurement.

Next, an arbitrary mass of the cooled carbonaceous material was taken (a mass at which the residual ratio of Reactive Black 5 in the filtrate was about 10% according to the following equation (II)) in a 100 mL stoppered Erlenmeyer flask, the carbonaceous material was added to 50 mL of the test solution A prepared above, and the mixture was shaken in water in a shaking thermostatic bath (water bath shaker MM-10 (trade name) manufactured by TAITEC CORPORATION) at a rate of 150 times/min and 40°C for 5 hours to obtain a mixed solution. The mixed solution was then filtered with a membrane filter (DISMIC (registered trademark) 25HP045AN (trade name) manufactured by TOYO ROSHI KAISHA, LTD.) to obtain a filtrate.

The absorbances of the obtained test solution B and the obtained filtrate were measured at 594 nm using a glass cell with an optical path length of 10 mm and an ultraviolet and visible spectrophotometer (double-beam spectrophotometer U-2910 (trade name) manufactured by Hitachi High-Tech Corporation). Using these absorbances, the adsorption amount of Reactive Black 5 per g of the carbonaceous material (hereinafter, simply referred to as "RB5 adsorption amount per g of carbonaceous material (/g)") was calculated by the following equation (I). 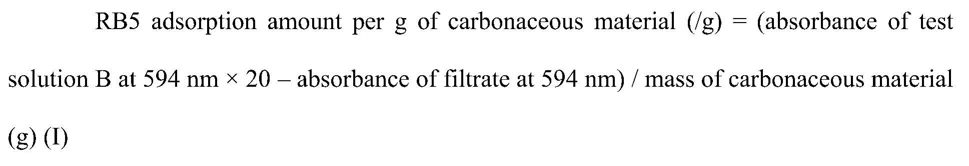 RB5 adsorption amount per g of carbonaceous material (/g) = (absorbance of test solution B at 594 nm × 20 - absorbance of filtrate at 594 nm) / mass of carbonaceous material (g) (I)

The residual ratio of Reactive Black 5 in the filtrate (hereinafter, simply referred to as "RB5 residual ratio (%)") was calculated by the following equation (II). 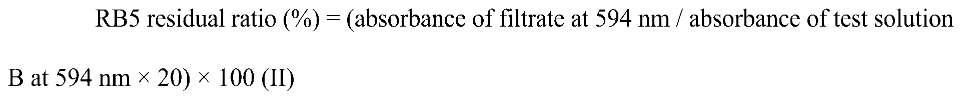 RB5 residual ratio (%) = (absorbance of filtrate at 594 nm / absorbance of test solution B at 594 nm × 20) × 100 (II)

Next, a power approximation curve was created with the RB5 residual ratio (%) on the horizontal axis and the RB5 adsorption amount per g of the carbonaceous material (/g) on the vertical axis. The adsorption amount of Reactive Black 5 when the Reactive Black 5 residual ratio was 1% (hereinafter, simply referred to as "RB5 adsorption amount (/g) at 1% RB5 residual ratio") was determined using the power-law approximation equation, and the Reactive Black 5 value (g/L) was calculated by the equation (III). 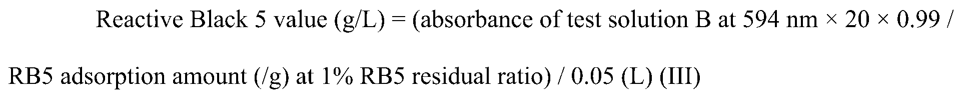 Reactive Black 5 value (g/L) = (absorbance of test solution B at 594 nm × 20 × 0.99 / RB5 adsorption amount (/g) at 1% RB5 residual ratio) / 0.05 (L) (III)

In the equation (III), 0.05 (L) is the amount of the test solution.

### (4) Iodine Adsorption Number (Iodine Adsorption Performance)

The iodine adsorption number (mg/g) of the carbonaceous material was measured and calculated.

Specifically, the iodine adsorption number was measured in accordance with JIS K 1474 (2014). First, according to JIS Z 8801-1, the carbonaceous material was pulverized until 90% or more of the carbonaceous material passed through a 45 µm mesh sieve, and then dried in a constant-temperature drier (DVS402 (trade name) manufactured by Yamato Scientific Co., Ltd.) at 115°C for 3 hours. Then, the dried product was allowed to cool to room temperature in a desiccator using silica gel as a drying agent to obtain a cooled carbonaceous material.

An iodine solution was prepared by dissolving 25.0 g of potassium iodide (manufactured by FUJIFILM Wako Pure Chemical Corporation) and 13.0 g of iodine (manufactured by FUJIFILM Wako Pure Chemical Corporation) in approximately 1 L of distilled water. The iodine solution was titrated with a 0.1 mol/L sodium thiosulfate solution (manufactured by FUJIFILM Wako Pure Chemical Corporation), and distilled water was appropriately added to the iodine solution to prepare a 0.05 mol/L iodine solution.

Thereafter, an arbitrary amount of the cooled carbonaceous material (an amount at which a residual iodine concentration in a supernatant of a filtrate described later was about 2.5 g/L) was weighed and placed in a 100 mL stoppered Erlenmeyer flask, and 50 mL of the 0.05 mol/L iodine solution was added with a volumetric pipette. The resulting mixture was shaken at 200 times/min for 15 minutes using a shaker (a medium-sized reciprocating shaker NR-10 (trade name) manufactured by TAITEC CORPORATION) at room temperature (20°C or higher and 30°C or lower) to cause the carbonaceous material to adsorb iodine. Thus, a mixed solution was obtained. The mixed solution was filtered with a mixed cellulose ester membrane filter (A045A025A (trade name) manufactured by TOYO ROSHI KAISHA, LTD.) to obtain a filtrate. Ten mL of a supernatant of the filtrate was collected with a volumetric pipette and titrated with a 0.1 mol/L sodium thiosulfate solution (manufactured by FUJIFILM Wako Pure Chemical Corporation, factor: 1.000), and the residual iodine concentration was calculated by the following equation (IV). 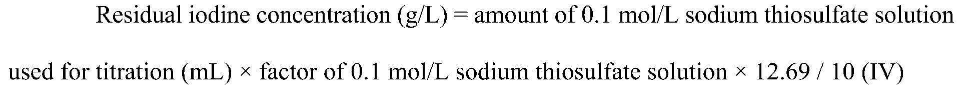 Residual iodine concentration (g/L) = amount of 0.1 mol/L sodium thiosulfate solution used for titration (mL) × factor of 0.1 mol/L sodium thiosulfate solution × 12.69 / 10 (IV)

The iodine adsorption number per g of the carbonaceous material was calculated by the following equation (V). 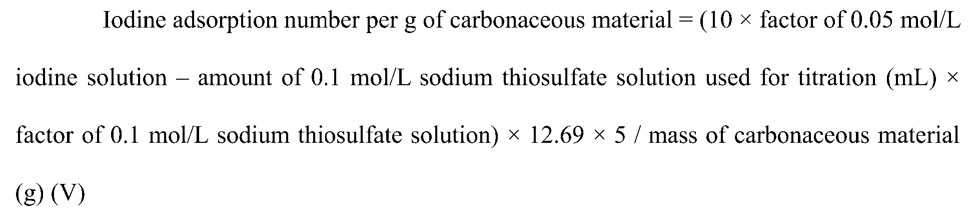 Iodine adsorption number per g of carbonaceous material = (10 × factor of 0.05 mol/L iodine solution - amount of 0.1 mol/L sodium thiosulfate solution used for titration (mL) × factor of 0.1 mol/L sodium thiosulfate solution) × 12.69 × 5 / mass of carbonaceous material (g) (V)

The factor of the 0.05 mol/L iodine solution was calculated by the equation (VI). 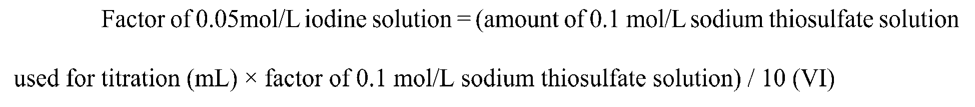 Factor of 0.05mol/L iodine solution = (amount of 0.1 mol/L sodium thiosulfate solution used for titration (mL) × factor of 0.1 mol/L sodium thiosulfate solution) / 10 (VI)

From the Freundlich equation, an adsorption isotherm was prepared with the residual iodine concentration on the horizontal axis and the iodine adsorption number per g of the carbonaceous material on the vertical axis, and the iodine adsorption number (mg/g) per g of the carbonaceous material at a residual iodine concentration of 2.5 g/L was calculated. The obtained iodine adsorption number was defined as the iodine adsorption performance.

### (5) Average Pore Size of Micropores

### · Determination of Specific Surface Area of Pores with Pore Size of 2.0 nm or less

The specific surface area (m²/g) of micropores with a pore size of 2.0 nm or less per g of the carbonaceous material was calculated as follows from the nitrogen adsorption isotherm obtained in the section "(1) Pore Volume of Pores with Pore Size of 0.80 nm or less" by the QSDFT method. Specifically, the pore size distribution was calculated by using the values of the nitrogen adsorption isotherm obtained in the above section "Determination of Nitrogen Gas Adsorption Isotherm with BELSORP-MAX" and applying N₂ at 77K carbon [slit pore / cyl. pore (QSDFT Ads. model)] as a calculation model, and thus the specific surface area (m²/g) of the micropores with a pore size of 2.0 nm or less was calculated.

### · Determination of Pore Volume of Pores with Pore Size of 2.0 nm or less

The pore volume (cm³/g) of micropores with a pore size of 2.0 nm or less per g of the carbonaceous material was calculated as follows from the nitrogen adsorption isotherm obtained in the section "(1) Pore Volume of Pores with Pore Size of 0.80 nm or less" by the QSDFT method. Specifically, the pore size distribution was calculated by using the values of the nitrogen adsorption isotherm obtained in the above section "Determination of Nitrogen Gas Adsorption Isotherm with BELSORP-MAX" and applying N₂ at 77K carbon [slit pore / cyl. pore (QSDFT Ads. model)] as a calculation model, and thus the pore volume (cm³/g) of the micropores with a pore size of 2.0 nm or less was calculated.

### · Method for Calculating Average Pore Size of Micropores

The average pore size (nm) of micropores of the carbonaceous material was calculated from the following equation (VII) using the specific surface area of the micropores and the pore volume of the micropores obtained above. 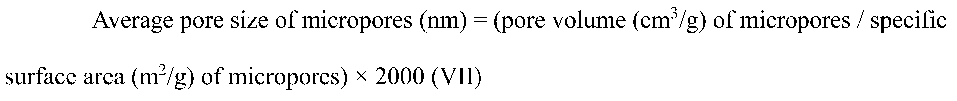 Average pore size of micropores (nm) = (pore volume (cm3/g) of micropores / specific surface area (m2/g) of micropores) × 2000 (VII)

### (6) Percentage of Pore Volume of Pores with Pore Size of 0.80 nm or less

The percentage (%) of the pore volume of pores with a pore size of 0.80 nm or less in the carbonaceous material was calculated.

Specifically, the percentage (%) of the pore volume of pores with a pore size of 0.80 nm or less in the carbonaceous material was calculated by the following equation (VIII) using the pore volume (cm³/g) of pores with a pore size of 0.80 nm or less obtained in the section of "(1) Pore Volume of Pores with Pore Size of 0.80 nm or less" and the pore volume (cm³/g) of pores with a pore size of 2.0 nm or less obtained in the section of "(5) Average Pore Size of Micropores." 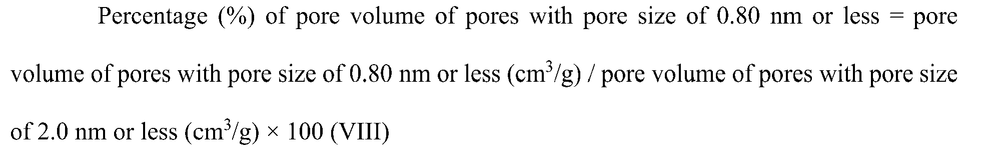 Percentage (%) of pore volume of pores with pore size of 0.80 nm or less = pore volume of pores with pore size of 0.80 nm or less (cm3/g) / pore volume of pores with pore size of 2.0 nm or less (cm3/g) × 100 (VIII)

### (8) n-Butane Adsorption Amount (n-Butane Adsorption Performance)

The n-butane adsorption amount of the carbonaceous material was measured and calculated.

Specifically, first, in accordance with JIS Z 8801-1, the carbonaceous material was sieved using a test sieve to obtain particles in the particle size range corresponding to a nominal aperture size of 0.600 mm or more and 0.250 mm or less. The carbonaceous material after sieving was dried in a constant-temperature drier (DVS402 (trade name), manufactured by Yamato Scientific Co., Ltd.) at 115±5°C for 3 hours. Thereafter, the carbonaceous material was allowed to cool to room temperature in a desiccator using silica gel as a drying agent to obtain a cooled carbonaceous material.

Next, a glass column (manufactured by Iwata Glass Industrial Co., Ltd.) with an inner diameter of 28 mm was filled with 0.492 g of the carbonaceous material. Thereafter, the glass column after filling was placed in a constant-temperature liquid tank (LF-681 (trade name), manufactured by ADVANTEC) kept at 25°C, and a water vapor gas adjusted to have a relative humidity of 50% RT was passed through the glass column at a flow rate of 7.39 L/min for 30 minutes to perform a pre-treatment.

The pretreated glass column was then placed in gas chromatograph (GC-2014 (trade name), manufactured by Shimadzu Corporation), and n-butane gas (concentration: 80 ppm) adjusted to have a relative humidity of 50% RT was passed through the column at a flow rate of 7.3 L/min as a test gas. While measuring the n-butane gas concentrations before and after passing through the column, the breakthrough ratio of the n-butane concentration (C) after passing through the column relative to the n-butane concentration (C₀) before passing through the column was calculated, and the test was performed until the value reached 95%. The breakthrough ratio was calculated by the following equation (IX). 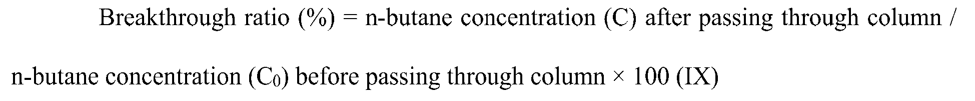 Breakthrough ratio (%) = n-butane concentration (C) after passing through column / n-butane concentration (C0) before passing through column × 100 (IX)

The n-butane adsorption amount (mg) per g of the carbonaceous material was calculated from the n-butane adsorption amount (mg) when the breakthrough ratio reached 95% and the packing amount of the carbonaceous material packed in the glass column using the following equation (X). The n-butane adsorption amount was defined as the n-butane adsorption performance. 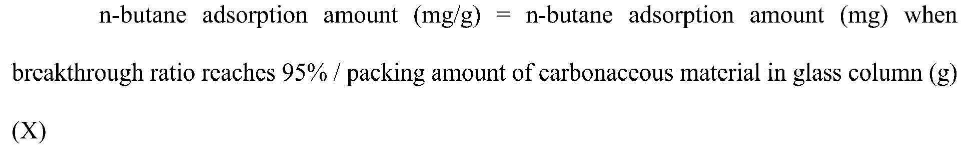 n-butane adsorption amount (mg/g) = n-butane adsorption amount (mg) when breakthrough ratio reaches 95% / packing amount of carbonaceous material in glass column (g) (X)

### [Example 1]

### (Carbonization)

Coconut shells from the Philippines were carbonized at a temperature of 600°C for about 2 hours, pulverized, and then adjusted to have a particle size (50% particle size in a volume-based cumulative distribution, D50) of 405 µm using a 70 mesh sieve (aperture size: 243 µm, manufactured by Nishimura Kanaami Seisakusho, Japan), on which particles are retained and a 14 mesh sieve (aperture size: 1.31 mm, manufactured by Nishimura Kanaami Seisakusho, Japan), through which particles are passed, in accordance with JIS Z 8801-1:2019 specifying test sieves of metal wire cloth, thereby obtaining a granulated carbide.

### (Activation Treatment)

The obtained carbide was charged into a fluidized bed furnace heated to 900°C, as shown in FIG. 1. Thereafter, an activating gas (water vapor: 20 vol%, oxygen: 2 vol%, carbon dioxide: 5 vol%, and nitrogen: 73 vol%) was introduced into the fluidized bed furnace, and an activation treatment was performed for 110 minutes to obtain an activated product.

### (Washing, Drying, and Other Steps)

The obtained activated product was sufficiently washed with water and dried to obtain a dried product. Thereafter, the obtained dried product was pulverized to obtain a pulverized carbonaceous material 1 as activated carbon.

### [Example 2]

A pulverized carbonaceous material 2 as activated carbon was obtained in the same manner as in Example 1 except that the activation treatment in the activation was performed for 130 minutes.

### [Example 3]

A pulverized carbonaceous material 3 as activated carbon was obtained in the same manner as in Example 1 except that the activation treatment in the activation was performed for 170 minutes.

### [Example 4]

A pulverized carbonaceous material 4 as activated carbon was obtained in the same manner as in Example 1 except that the activation treatment in the activation was performed for 190 minutes.

### [Example 5]

A pulverized carbonaceous material 5 as activated carbon was obtained in the same manner as in Example 1 except that the activation treatment in the activation was performed for 205 minutes.

### [Example 6]

A pulverized carbonaceous material 6 as activated carbon was obtained in the same manner as in Example 1 except that the activation treatment in the activation was performed for 225 minutes.

### [Example 7]

A pulverized carbonaceous material 7 as activated carbon was obtained in the same manner as in Example 1 except that the activation treatment in the activation was performed for 90 minutes.

### [Example 8]

A pulverized carbonaceous material 8 as activated carbon was obtained in the same manner as in Example 1 except that the activation treatment in the activation was performed for 240 minutes.

### [Example 9]

A pulverized carbonaceous material 9 as activated carbon was obtained in the same manner as in Example 1 except that the activation treatment in the activation was performed for 300 minutes.

### [Comparative Example 1]

### (Carbonization)

Coconut shells from the Philippines were carbonized at a temperature of 600°C for about 2 hours, pulverized, and then adjusted to have a particle size (50% particle size in a volume-based cumulative distribution, D50) of 405 µm using a 70 mesh sieve (aperture size: 243 µm, manufactured by Nishimura Kanaami Seisakusho, Japan), on which particles are retained and a 14 mesh sieve (aperture size: 1.31 mm, manufactured by Nishimura Kanaami Seisakusho, Japan), through which particles are passed, in accordance with JIS Z 8801-1:2019 specifying test sieves of metal wire cloth, thereby obtaining a granulated carbide.

### (Activation Treatment)

The obtained carbide was placed in a rotary kiln having stirring blades in a furnace heated to 900°C in an amount of about 0.06 times the volume of 1 m³ of the rotary kiln. Thereafter, while rotating the kiln at a rotation speed of 3.0 rpm, an activating gas (water vapor: 35 vol%, oxygen: 5 vol%, carbon dioxide: 5 vol%, and nitrogen: 55 vol%) was introduced into the kiln, and then taken out from the kiln immediately after the introduction, thereby obtaining an activated product.

### (Washing, Drying, and Other Steps)

The obtained activated product was sufficiently washed with water and dried to obtain a dried product. Thereafter, the obtained dried product was pulverized to obtain a pulverized carbonaceous material 10 as activated carbon.

### [Comparative Example 2]

A pulverized carbonaceous material 11 as activated carbon was obtained in the same manner as in Comparative Example 1 except that the activation treatment in the activation was performed for 170 minutes.

### [Comparative Example 3]

A pulverized carbonaceous material 12 as activated carbon was obtained in the same manner as in Comparative Example 1 except that the activation treatment in the activation was performed for 220 minutes.

### [Comparative Example 4]

### (Carbonization)

Wood flour from Japan and Malaysia were carbonized at a temperature of 600°C for about 2 hours, pulverized, and then adjusted to have a particle size (50% particle size in a volume-based cumulative distribution, D50) of 1451 µm using a 20 mesh sieve (aperture size: 870 µm, manufactured by Nishimura Kanaami Seisakusho, Japan), on which particles are retained and a 10 mesh sieve (aperture size: 1.54 mm, manufactured by Nishimura Kanaami Seisakusho, Japan), through which particles are passed, in accordance with JIS Z 8801-1:2019 specifying test sieves of metal wire cloth, thereby obtaining a granulated carbide.

### (Activation Treatment)

The obtained carbide was placed in a rotary kiln having stirring blades in a furnace heated to 850°C in an amount of about 0.06 times the volume of 1 m³ of the rotary kiln. Thereafter, while rotating the kiln at a rotation speed of 3.0 rpm, an activating gas (water vapor: 50 vol%, oxygen: 10 vol%, carbon dioxide: 35.0 vol%, and nitrogen: 5.0 vol%) was introduced into the kiln, and an activation treatment was performed until the specific surface area reached 1080 m²/g, thereby obtaining an activated product.

### (Washing, Drying, and Other Steps)

The obtained activated product was washed with diluted hydrochloric acid, sufficiently washed with water to remove residual hydrochloric acid, and dried to obtain a dried product. Thereafter, the obtained dried product was pulverized to obtain a pulverized carbonaceous material 13 as activated carbon.

### [Comparative Example 5]

The granulated carbide obtained in Example 1 was subjected to the activation treatment by changing the activation time from 110 minutes to 225 minutes in the activation of Example 1. The obtained activated product was pulverized, and then adjusted to have a particle size (50% particle size in a volume-based cumulative distribution, D50) of 334 µm using a 60 mesh sieve (aperture size: 243 µm, manufactured by Nishimura Kanaami Seisakusho, Japan), on which particles are retained and a 30 mesh sieve (aperture size: 550 µm, manufactured by Nishimura Kanaami Seisakusho, Japan), through which particles are passed, in accordance with JIS Z 8801-1:2019 specifying test sieves of metal wire cloth, thereby obtaining a granulated activated product.

Thereafter, the obtained granulated activated product was immersed in 0.5N hydrochloride preheated to 95°C, and allowed to stand at 95°C for 20 minutes while heating. Next, the activated product after immersion was washed with water until the pH reached about 6 to about 7, and then dried until the water content of the activated product reached 3 mass% or less, thereby obtaining dried activated carbon. The specific surface area of the dried active carbon was 1,050 m²/g. Next, a 18 mass% aqueous solution of sulfanilic acid (sulfanilic acid: special grade reagent, manufactured by FUJIFILM Wako Pure Chemical Corporation, an aqueous solution containing 0.95 mol of sodium hydroxide relative to 1 mol of sulfanilic acid) was sprayed onto the dried activated carbon to impregnate activated carbon with sulfanilic acid, thereby obtaining an impregnated product 1. The obtained impregnated product 1 was left as it was at room temperature for 30 minutes, and then a 46 mass% aqueous solution of citric acid was sprayed to impregnate the activated carbon with citric acid, thereby obtaining an impregnated product 2. The obtained impregnated product 2 was left as it was at room temperature for 60 minutes, thereby obtaining impregnated carbon. Next, the impregnated carbon was dried in a constant-temperature dryer (DVS402 (trade name), manufactured by Yamato Scientific co., ltd.) at 90°C for 24 hours, thereby obtaining a carbonaceous material 14, which was impregnated carbon (containing 8.0 parts by mass of sulfanilic acid and 4.4 parts by mass of citric acid relative to 100 parts of the activated carbon).

**[Table 1]**

| | Carbonaceous material | Pore volume of pores with pore size of 0.80 nm or less | Bulk density | Reactive Black 5 value | Iodine adsorption performance | Percentage of pore volume of pores with pore size of 0.80 nm or less | Average pore size of micropores | n-butane adsorption performance |
|---|---|---|---|---|---|---|---|---|
| | | (cm³/g) | (g/mL) | (g/L) | (mg/g) | (%) | (nm) | (mg/g) |
| Example 1 | 1 | 0.23 | 0.59 | 46.8 | 821 | 88 | 0.63 | 44.3 |
| Example 2 | 2 | 0.24 | | 40.0 | 859 | 86 | 0.64 | 45.7 |
| Example 3 | 3 | 0.26 | 0.56 | 28.0 | 946 | 82 | *0.65* | 46.5 |
| Example 4 | 4 | 0.27 | 0.54 | 21.1 | 1015 | 79 | 0.66 | 46.0 |
| Example 5 | 5 | 0.27 | 0.53 | 20.0 | 1028 | 78 | 0.66 | 46.2 |
| Example 6 | 6 | 0.28 | 0.52 | 15.4 | 1093 | 75 | 0.68 | 44.7 |
| Example 7 | 7 | 0.23 | | 51.2 | 799 | 89 | 0.63 | 42.8 |
| Example 8 | 8 | 0.28 | 0.52 | 14.0 | 1115 | 73 | 0.68 | 42.6 |
| Example 9 | 9 | 0.29 | 0.55 | 18.8 | 1043 | 81 | 0.67 | 41.7 |
| Comparative Example 1 | 10 | 0.22 | 0.54 | 60.8 | 700 | 93 | 0.62 | 27.0 |
| Comparative Example 2 | 11 | 0.28 | 0.42 | 5.0 | 1367 | 60 | 0.74 | 33.2 |
| Comparative Example 3 | 12 | 0.24 | 0.26 | 1.9 | 1600 | 46 | 0.81 | 16.1 |
| Comparative Example 4 | 13 | 0.28 | 0.18 | 2.8 | 1103 | 82 | 0.68 | 8.5 |
| Comparative Example 5 | 14 | 0.18 | 0.54 | 16.9 | 800 | 73 | 0.61 | 15.9 |

### INDUSTRIAL APPLICABILITY

The carbonaceous material of the present embodiment can be suitably used for applications for removal, adsorption, concentration, and recovery of butanes.

The present application is based on Japanese patent application filed on November 29, 2023 (Japanese Patent Application No. 2023-201660), the contents of which are incorporated herein by reference.

### DESCRIPTION OF REFERENCE CHARACTERS

- A: Main Direction of Gas
- 1, 2, 3, 4: Gas Inlet
- 5: Fluidized Bed
- 6: Combustion Layer of Combustible Gas
- 7: Oxygen-Containing Gas Inlet
- 8: Gas Outlet

## Claims

1. A carbonaceous material having
a pore volume (cm³/g) of 0.23 cm³/g or more and 0.35 cm³/g or less per g of the carbonaceous material for pores with a pore size of 0.80 nm or less, the pore volume being calculated from a nitrogen adsorption isotherm by a QSDFT method,
a bulk density of 0.43 g/mL or more and 0.65 g/mL or less measured in accordance with JIS K 1474 (2014), and
a Reactive Black 5 value of 3.0 g/L or more and 60.0 g/L or less.

2. The carbonaceous material of claim 1, wherein
an iodine adsorption number is 710 mg/g or more and 1,500 mg/g or less.

3. The carbonaceous material of claim 1, wherein
a percentage of the pore volume of pores with a pore size of 0.80 nm or less is 61% or more and 92% or less.

4. The carbonaceous material of claim 1, wherein
an average pore size of micropores is 0.60 nm or more and 0.80 nm or less.

5. The carbonaceous material of any one of claims 1 to 4, wherein
the carbonaceous material is used for adsorbing at least one selected from the group consisting of n-butane, isobutane, 1,2-butadiene, and 1,3-butadiene.

6. A method for producing the carbonaceous material of any one of claims 1 to 4, the method comprising:
carbonizing a raw material to obtain a carbide; and
subjecting the carbide to an activation treatment to obtain an activated product.

7. The method of claim 6, further comprising:
washing the activated product.

8. The method of claim 6, wherein
the raw material is coconut shells.

9. An adsorption filter comprising:
the carbonaceous material of any one of claims 1 to 4.

10. The adsorption filter of claim 9, wherein
the carbonaceous material is used for an automobile.
